(19) Europäisches Patentamt
European Patent Office
Office européen des brevets

(11) Publication number: 0 156 728 B1

(12) **EUROPEAN PATENT SPECIFICATION**

(45) Date of publication of patent specification: **02.11.88**

(51) Int. Cl.[4]: **C 07 C 155/02,** C 07 C 154/02

(21) Application number: **85400544.4**

(22) Date of filing: **21.03.85**

(54) **Process for the preparation of thiocarbamates.**

(30) Priority: **22.03.84 IT 2017484**

(43) Date of publication of application:
**02.10.85 Bulletin 85/40**

(45) Publication of the grant of the patent:
**02.11.88 Bulletin 88/44**

(84) Designated Contracting States:
**BE DE FR GB NL**

(56) References cited:
**US-A-4 222 938**

**SYNTHESIS, no. 7, July 1983, pages 630-632, Stuttgart, DE; I. DEGANI et al.: "S,S-Dialkyl dithiocarbonates as a convenient source of alkanethiolate anions in phase-transfer-catalysis systems: An improved synthesis of organic sulfides"**

(73) Proprietor: **Montedison S.p.A.**
**31, Foro Buonaparte**
**I-20121 Milan (IT)**

(72) Inventor: **Ugo, Renato**
**33 Via Nino Bixio**
**I-20 129 Milan (IT)**
Inventor: **Campolmi, Stefano**
**27 via Andrea Costa**
**F-28100 Novara (IT)**
Inventor: **Carletti, Vittorio**
**7 Largo Europa**
**I-20 036 Meda Milan (IT)**

(74) Representative: **Hirsch, Marc-Roger**
**Cabinet Hirsch 34 rue de Bassano**
**F-75008 Paris (FR)**

**EP 0 156 728 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a new process for the preparation of organic thiocarbamates having the following general formula:

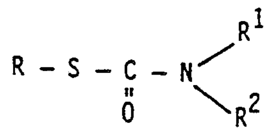

where:

R represents a $C_1$—$C_4$ alkyl optionally substituted with one or more halogen atoms, a $C_2$—$C_4$ alkenyl optionally substituted with one or more halogen atoms, a phenyl optionally substituted with one or more halogen atoms, a benzyl optionally substituted with one or more halogen atoms in the phenyl portion;

$R^1$ and $R^2$, equal or different from each other, represent a $C_1$—$C_4$ alkyl, a cycloalkyl.

It is known that thiocarbamates constitute a wide class of industrial products used in different fields of "fine chemicals", particularly as herbicides and fungicides.

Among them can be cited Eptam®, Pebulate®, Cycloate®, Butylate®, Vernolate® and Molinate® manufactured by Stauffer, Drepamon® by Montedison, Diallate® and Triallate® by Monsanto, Benthiocarb® and Orbencarb® by Kumiai, Methiobencarb® by Bayer, Prothiocarb® by Schering; all of which products have the following general formula:

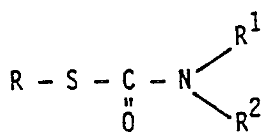

where:

R, $R^1$ and $R^2$ have different meanings for each single product.

Due to the importance of this class of products, several processes have been proposed up until now to prepare them.

From among these, the presently known process with widest industrial use considers the reaction between a mercaptide and a carbamoil chloride, according to the scheme:

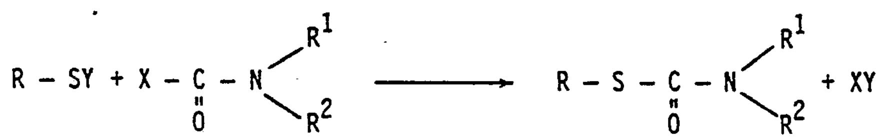

where:

R, $R^1$, $R^2$ have different meanings;

X is a halogen; and

Y is an alkaline metal.

The main disadvantage of this process lies in the use of mercaptans R—SH, which, as it is known, are toxic, foul smelling and volatile compounds.

Are also known from the prior art the following publications: Synthesis, N 7; 630—632; (1983) I. Degani et al. which discloses that symmetrical S,S-dialkyldithiocarbonates, easily obtainable by an ammonium salt-catalyzed rearrangement of corresponding O,S-dialkyldithiocarbonates, readily react with electrophiles, such as organic halides, to give organic sulfides, in the presence of a quaternary ammonium or phosphonium salt under phase transfer conditions; US—A—4 222 938 which discloses the preparation of thiolcarbamates by reacting a carbamoil halide with a xanthate according to the following reaction scheme:

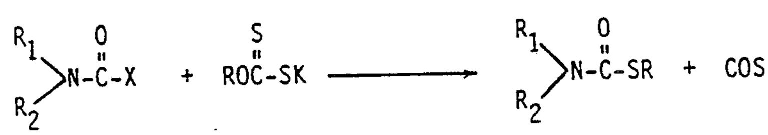

where

R, $R_1$, $R_2$ have different meanings

X is a halogen

The main disadvantage of this process lies in the fact that a toxic, foul smelling and volatile carbonyl sulfide (COS) is obtained as a by product of the reaction.

A new process, which is both simple and economic, has now been found and it forms the object of the present invention; it allows to prepare on an industrial scale, with high yields, the above thiocarbamates.

This process is a general one and can be used for the preparation of any thiocarbamate included in the aforementioned formula, under substantially equivalent conditions.

The new process is characterized by the following reactions (I) and (II):

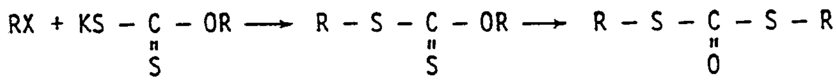 (I)

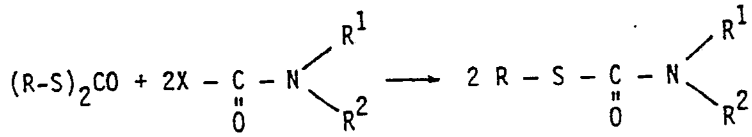 (II)

where:

X is a halogen;

R, $R^1$ and $R^2$ have the above-mentioned meanings,

said reactions (I) and (II) being carried out in sequence without isolation of intermediate products, in an alkaline aqueous-organic medium, in the presence of a catalyst for phase transfer, the xanthogenate/catalyst molar ratios ranging from 20:1 to 10:1 and the alkaline base/xanthogenate molar ratios from 4:1 to 10:1.

Reaction (I) comprises two steps: the first one of preparing O,S-dialkyldithiocarbonates from organic halides and potassium xanthogenates (the latter obtainable successively reacting carbonium sulfide, KOH and the corresponding alcohol), under conditions of phase transfer, and the second one of transposing O,S-dialkyldithiocarbonates into S,S-dialkyldithiocarbonates, always through phase transfer catalysis.

As both steps forming reaction (I) are catalyzed by the same onium salts, they can practically be performed in sequence.

Reaction (II) must be carried out in the presence of an aqueous alkaline base and of a phase transfer catalyst, at a temperature of between 10 and 50°C, for time periods varying according to the products reacted, but in general ranging from between 5 to 10 total hours.

The initial products are reacted in the stoichiometric ratios required by reaction (II). When the reaction is completed, the resulting organic phase is essentially formed by raw thiocarbamate, with possibly the catalyst if liposoluble; the thiocarbamate can be isolated either simply by decantation, if liquid, or by filtration, if solid, then it can be furtherly purified, if necessary, according to conventional techniques.

In this way, the process can be carried out in the absence of organic solvents.

The preferably used aqueous bases for obtaining reaction (II) are NaOH and 30% KOH. In this case, the reaction scheme is represented by the following equation:

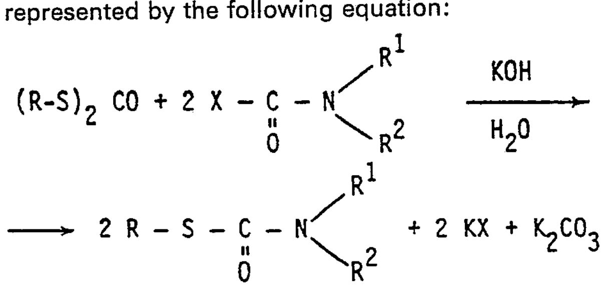


(II)

Commonly known phase transfer catalysts can be used as suitable catalysts to perform the process according to the present invention, in particular "onium" salts with formula: $Q(R')_4Y$, where Q is an atom of N, P, As, Sb; R' represents an alkyl radical containing up to 20 C atoms; Y is a halogen selected from among Cl, Br, I; the preferred salts are ammonium salts selected from the group comprising tetrabutylammonium bromide, tricaprylmethylammonium chloride (Aliquat 336), trilaurylmethylammonium chloride, and phosphonium salts selected from tetrabutylphosphonium chloride and bromide. Also crown ethers can be used as phase transfer catalysts, such as dicyclohexyl-18-crown-6 and dibenzo-18-crown-6.

The preparation of dithiocarbonates to be used as starting products in reaction (II), according to reaction (I), is advantageous, in that reactions (I) and (II), using the same catalysts, can be practically carried out in sequence in a single reactor without isolation of intermediate products. In this case, the xanthogenate/catalyst molar ratios are selected within an interval ranging from 20:1 to 10:1, while the alkaline base/xanthogenate molar ratios are selected within an interval ranging from 4:1 to 10:1.

The process will now be described in further detail in the following examples, given by way of non-limitative illustration.

Example 1

Preparation of potassium xanthogenate

13.2 g of 85% KOH in lozenges and 100 cc of benzyl alcohol are placed in a 500 cc flask equipped with a mechanical stirrer, thermometer, reflux condenser, dripping funnel, nitrogen inlet and bubble counter at

the outlet; heating is carried out at 70—80°C for half an hour until KOH has dissolved completely. Cooling is performed at approximately 25°C, then 15.2 g of carbonium sulfide are dripped during 15—20 minutes, keeping the temperature below 40°C. Stirring is continued for about 1 hour, then approximately 200 cc of ethyl ether are introduced, the precipitated product is filtered and dried. 43.8 g of potassium benzyl xanthogenate are obtained (yield = 98.6%).

Preparation of S-benzyl di-sec-butylthiocarbamate (Drepamon)

2.44 g of potassium benzyl xanthogenate (0.011 moles of the previous preparation), 0.3 g approx. of Aliquat 336 (0.00007 moles), 1.26 g of benzyl chloride (0.01 moles) and 12 cc of water are placed in a 50 cc flask provided with magntic stirring, thermometer, nitrogen inlet. Stirring is continued at 25°C for about 12 minutes, then at 40°C for 2 hours. Cooling is then carried out and 6 g of 85% KOH in lozenges (0.09 moles) are introduced keeping the temperature at 25°C; 4.13 g of di-sec-butyl-carbamoil chloride (0.022 moles) are finally added and the reaction is continued for 8—10 hours.

When the reaction is completed, the organic phase is extracted with ethyl ether and gas chromatographically analyzed (n-decane as internal standard). 4.78 g of S-benzyl di-sec-butylthiocarbamate (Drepamon) (0.017 moles) are found with 77.3% yield on carbamoil halide.

**Claims**

1. Process for the preparation of thiocarbamates having the general formula:

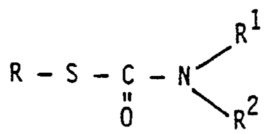

where:

R represents a $C_1$—$C_4$ alkyl optionally substituted with one or more halogen atoms, a $C_2$—$C_4$ alkenyl optionally substituted with one or more halogen atoms, a phenyl optionally substituted with one or more halogen atoms, a benzyl optionally substituted with one or more halogen atoms in the phenyl portion;

$R^1$ and $R^2$, equal or different from each other, represent a $C_1$—$C_4$ alkyl, a cycloalkyl,

according to the following reactions (I) and (II):

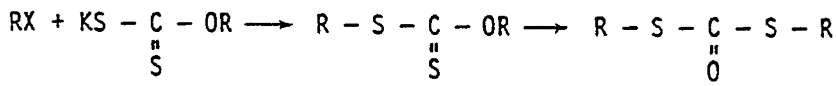 (I)

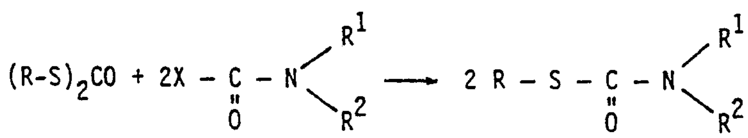 (II)

where:

X is a halogen;

R, $R^1$ and $R^2$ have the above-mentioned meanings,

wherein reactions (I) and (II) are carried out in sequence without isolation of intermediate products, in alkaline aqueous-organic medium, in the presence of a catalyst for phase transfer, with xanthogenate/catalyst molar ratios ranging from 20:1 to 10:1 and alkaline base/xanthogenate molar ratios from 4:1 to 10:1.

2. Process according to claim 1, wherein organic dithiocarbonate and carbamoil halide of reaction (II) are reacted in stoichiometric ratios (1:2), at a temperature between 10° and 50°C, for a time period ranging from 5 to 10 hours, in the presence of an aqueous solution selected from among NaOH and KOH and in the presence of a catalyst for phase transfer.

3. Process according to claims 1 and 2, wherein the catalyst for phase transfer is an "onium" salt having formula $Q(R')_4Y$, where Q is an atom of N, P, As and Sb; R' represents an alkyl radical containing up to 20 C atoms; Y is a halogen selected from among chlorine, bromine and iodine.

4. Process according to claim 3, wherein the "onium" salt is selected from the group consisting of tetrabutylammonium bromide, tricaprylmethylammonium chloride, trilaurylmethylammonium chloride, tetrabutylphosphonium chloride and bromide.

5. Process according to claims 1 and 2, wherein the catalyst for phase transfer is a crown ether compound selected from the group consisting of dicyclohexyl-18-crown-6 and dibenzo-18-crown-6.

6. Process according to claim 1, wherein thiocarbamate obtained is formed by S-benzyl di-sec-butylthiocarbamate.

**Patentansprüche**

1. Verfahren zur Herstellung von Thiokarbamaten mit der allgemeinen Formel:

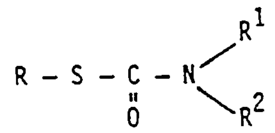

worin:

R ein ggf. mit einem oder mehreren Halogenatomen substituiertes Alkyl mit 1 bis 4 Kohlenstoffatomen, ein ggf. mit einem oder mehreren Halogenatomen substituiertes Alkenyl mit 2 bis 4 Kohlenstoffatomen, ein ggf. mit einem oder mehreren Halogenatomen substituiertes Phenyl oder ein ggf. mit einem oder mehreren Halogenatomen im Phenylanteil substituiertes Benzyl darstellt, während

$R^1$ und $R^2$, die gleich oder verschieden sein können, ein Alkyl mit 1 bis 4 Kohlenstoffatomen oder ein Zykloalkyl darstellen,
gemäss nachstehender Reaktionen (I) und (II):

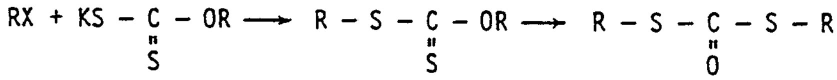 (I)

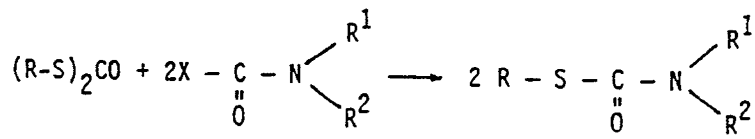 (II)

worin:

X ein Halogen darstellt;

R, $R^1$ und $R^2$ die vorstehend angegebene Bedeutung haben; wobei die Reaktionen (I) und (II) nacheinander ohne Abscheidung von Zwischenprodukten in einem alkalischen, organischen wässerigen Medium und in Anwesenheit eines Phasenüberführungskatalysators derart durchgeführt werden, dass das molare Xanthogenat/Katalysator-Verhältnis zwischen 20/1 und 10/1 und das molare Alkalibase/Xanthogenat-Verhältnis zwischen 4/1 und 10/1 liegt.

2. Verfahren nach Anspruch 1, bei welchem das organische Dithiokarbonat und das Karbamoylhalogenid der Reaktion (II) in stöchiometrischen Mengen (1/2) bei einer Temperatur von 10° bis 50°C miteinander zur Reaktion gebracht werden, und zwar während 5 bis 10 Stunden und in Gegenwart einer wässerigen Lösung von NaOH oder KOH, sowie in Gegenwart eines Phasenüberführungskatalysators.

3. Verfahren nach Anspruch 1 und 2, bei welchem der Phasenüberführungskatalysator ein "-onium"-Salz mit der Formel $Q(R')_4Y$ ist, worin Q ein N-, P-, As- bzw. Sb-Atom, R' ein höchstens 20 Kohlenstoffatome enthaltendes Alkylradikal und Y ein Halogen der Gruppe Chlor, Brom und Jod darstellt.

4. Verfahren nach Anspruch 3, bei welchem das "-onium"-Salz zu der Tetrabutylammoniumbromid, Tricaprylmethylammoniumchlorid, Trilaurylmethylammoniumbromid, Tetrabutylphosphoniumchlorid und Tetrabutylphosphoniumbromid unfassenden Gruppe gehört.

5. Verfahren nach Anspruch 1 und 2, bei welchem der Phasenüberführungskatalysator eine "Kron"-ätherverbindung der Dizyklohexyl-18-Kron-6 und Dibenzo-18-Kron-6 umfassenden Gruppe ist.

6. Verfahren nach Anspruch 1, bei welchem das erzielte Thiokarbamat S-benzyl-di-sec-butylthiokarbamat ist.

**Revendications**

1. Procédé de préparation de thiocarbamates de formule générale:

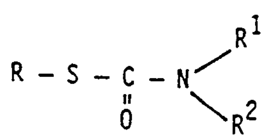

où

R représente un alkyle en $C_1$ à $C_4$, éventuellement substitué par un ou plusieurs atomes d'halogène, un alkényle en $C_2$ à $C_4$ éventuellement substitué par un ou plusieurs atomes d'halogène, un phényle éventuellement substitué par un ou plusieurs atomes d'halogène ou un benzyle dont la partie phényle est éventuellement substituée par un ou plusieurs atomes d'halogène;

$R^1$ et $R^2$, identiques ou différents, représentent un alkyle en $C_1$ à $C_4$ ou un cycloalkyle; selon les réactions (I) et (II) suivantes:

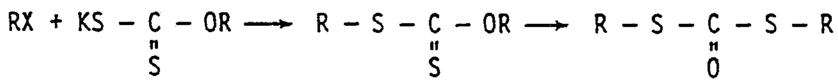 (I)

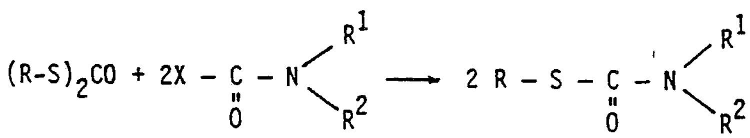 (II)

où:

X est un halogène;

R, $R^1$ et $R^2$ ont les significations indiquées ci-dessus;

procédé dans lequel on conduit lesdites réactions (I) et (II) consécutivement sans séparation des produits intermédiaires, dans un milieu organique aqueux alcalin, en présence d'un catalyseur de transfert de phase, avec des rapports molaires xanthogénate/catalyseur compris entre 20/1 et 10/1 et des rapports molaires base alcaline/xanthogénate de 4/1 à 10/1.

2. Procédé selon la revendication 1, dans lequel le dithiocarbonate organique et l'halogénure de carbamoyl résultant de la réaction (II) sont amenés à réagir l'un avec l'autre dans des proportions stoechiométriques (1/2) à une température comprise entre 10° et 50°C pendant 5 à 10 heures, en présence d'une solution aqueuse de NaOH ou de KOH et en présence d'un catalyseur de transfert de phase.

3. Procédé selon la revendication 1 et 2, dans lequel le catalyseur de transfert de phase est un sel "-onium" de formule $Q(R')_4Y$ où Q est un atome de N, P, As ou Sb, cependant que R' représente un radical alkyle renfermant jusqu'à 20 atomes de carbone et que Y est un halogène consistant en chlore, brome et iode.

4. Procédé selon la revendication 3, dans lequel ledit sel "-onium" est choisi parmi ceux formant le groupe comprenant le bromure de tétrabutylammonium, le chlorure de tricaprylméthylammonium, le chlorure de trilaurylméthylammonium, le chlorure de tétrabutylphosphonium et le bromure de tétrabutylphosphonium.

5. Procédé selon la revendications 1 et 2, dans lequel le catalyseur de transfert de phase est un éther "couronne" consistant en dicyclohexyl-18-couronne-6 ou dibenzo-18-couronne-6.

6. Procédé selon la revendication 1, dans lequel le thiocarbamate obtenu est du S-benzyl-di-sec-butylthiocarbamate.